# FASCICULE DE BREVET EUROPEEN

(11) **EP 0 862 910 B1**
(45) Date de publication et mention de la délivrance du brevet: **02.05.2003**
(21) Numéro de dépôt: 98400256.8
(22) Date de dépôt: 05.02.1998
(51) Int. Cl.: A61K 7/42, A61K 7/48

(54) **Utilisation de rétinoides en tant qu'agent induisant la pigmentation**
Verwendung von Retinoiden zur Induzierung von Hautpigmentierung
Use of retinoids as skin pigmentation inducing agent

(30) Priorité: 10.02.1997 FR 9701500
(43) Date de publication de la demande: 09.09.1998
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: Diaz, Philippe, 241, Route de Saint-Antoine, 06200 Nice (FR); Charpentier, Bruno, 06410 Biot (FR); Shroot, Braham, 06600 Antibes (FR)
(74) Mandataire: Tezier Herman, Béatrice

(56) Documents cités:
- EP-A- 0 199 636
- EP-A- 0 465 343
- EP-A- 0 509 421
- WO-A-93/19729

## Description

L'invention concerne l'utilisation, dans une composition cosmétique ou pour la préparation d'une composition pharmaceutique pour favoriser la pigmentation de la peau et/ou de ses phanères, d'au moins un rétinoïde portant une fonction phénol ou naphtol. Elle concerne également un procédé cosmétique pour pigmenter la peau et/ou ses phanères, caractérisé en ce que l'on applique sur la peau et/ou ses phanères une composition comprenant au moins un rétinoïde portant une fonction phénol ou naphtol.

La couleur de la peau humaine et de ses phanères (cheveux, ongles, poils, ...) est fonction de différents facteurs et notamment des saisons de l'année, de la race, du sexe et de l'âge. Elle est principalement déterminée par la concentration de mélanine produite par les mélanocytes. Les mélanocytes sont les cellules spécialisées qui, par l'intermédiaire d'organelles particuliers, les mélanosomes, synthétisent la mélanine.

On sait que dans la plupart des populations la coloration brune de la peau ou le maintien d'une coloration constante de la chevelure sont des aspirations importantes.

Il existe par ailleurs des maladies de la pigmentation comme par exemple le vitiligo qui est une maladie auto-immune qui se caractérise par l'apparition de plaques blanches sur la peau liées à un défaut de pigmentation.

Il existe donc un réel besoin de produit facilitant et/ou améliorant la pigmentation de la peau et/ou de ses phanères, plus particulièrement les cheveux et les ongles.

A cet égard, il a été proposé de nombreuses solutions dans le domaine de la coloration artificielle, par apport de colorants exogènes sensés donner à la peau et/ou aux cheveux une coloration la plus proche possible de ce qu'elle est naturellement ou dans le domaine de la coloration naturelle par stimulation des voies naturelles de pigmentation.

D'excellents résultats sont certes obtenus par les solutions proposées dans l'art antérieur, mais il n'en demeure pas moins que la stimulation de la pigmentation de la peau ou de ses phanères par la voie naturelle (mélanogénèse) reste la voie idéale de pigmentation.

A cet égard, il a été proposé dans les documents WO-A-9517161, WO-9511003, WO-A-9501773, WO-A-9404674, WO-A-9404122, EP-A-585018, WO-A-9310804, WO-A-9220322 ou WO-A-9107945 des solutions aussi variées que des compositions contenant un inhibiteur de phosphodiestérases, l'utilisation de prostaglandine, de fragments d'ADN, de dérivés de la tyrosine ou encore d'extraits de plantes. Souvent les composés utilisés sont des mélanges complexes qui ne présentent pas de spécificité.
La découverte de substances ayant un effet sur la pigmentation de la peau ou de ses phanères reste un objectif majeur de la recherche dans ce domaine.

Un des buts de la présente invention est donc de proposer l'utilisation de nouveaux composés pour favoriser la pigmentation de la peau et/ou de ses phanères.

La demanderesse a maintenant découvert que certains rétinoïdes induisent la mélanogénèse et donc favorisent la pigmentation de la peau ou de ses phanères. Ceci est d'autant plus surprenant que d'autres rétinoïdes ont été décrits comme dépigmentants. Ainsi, l'acide rétinoïque *tout-trans* a été décrit comme agent capable d'éclaircir les tâches hyperpigmentées de la peau (J.S. Weiss et al. "Topical tretinoin improves photodamaged skin", J.Am. Med.Assoc, 1988, 259, 527-532. Voir aussi EP 0 465 343. De plus, l'association de dérivés de vitamines A et d'antioxydants, comme des quinones ou des dérivés flavonoïques, était connue pour réduire les taux de mélanine à la fois dans la peau hyperpigmentée et dans la peau normale (U.S. 3856934, EP 421110, JP 60-48934, JP 63-301810).

Les documents brevets WO-A-93/19729 et EP-A-0 509 421 décrivent l'utilisation de certains composés de type rétinoide comme agents pigmentants.

Le document brevet EP-A-0 199 636 décrit certains composés de type rétinoide portant une fonction naphtol ou phénol et leur utilisation générale en dermatologie.

Ainsi, l'invention a pour objet l'utilisation dans une composition cosmétique ou pour la préparation d'une composition pharmaceutique, à titre de principe actif, d'une quantité suffisante d'au moins un rétinoïde portant une fonction phénol ou naphtol, ce rétinoïde ou la composition étant destiné à favoriser la pigmentation de la peau et/ou de ses phanères.

Elle concerne également un procédé cosmétique pour pigmenter la peau et/ou ses phanères, caractérisé en ce que l'on applique sur la peau et/ou ses phanères une composition comprenant, dans un support cosmétiquement acceptable, au moins un rétinoïde portant une fonction phénol ou naphtol. Un autre objet de l'invention concerne une composition cosmétique ou pharmaceutique comprenant, dans un support physiologiquement acceptable, au moins un rétinoïde portant une fonction phénol ou naphtol et au moins un substrat d'au moins une enzyme présentant une activité tyrosinase.

Parmi les phanères, on préfère les cheveux ou les ongles.

Par rétinoïde portant une fonction phénol ou naphtol, on entend des composés qui présentent à la fois:
- une activité biologique de type rétinoïde, c'est à dire jouant sur la prolifération et la différentiation cellulaire, comme cela a été défini dans L.J. Gudas et al, 1994, pp 443-520, chapitre 11, Cellular biology and biochemistry of the retinoids, In the Retinoids, Biology, Chemistry and Medecine et dans Sporn, M.B. Roberts, A.B., Goodman, D.S., Eds. Raven Press; Pawson et al. Retinoids at the threshold : their biological significance and therapeutic potential. J. Med. Chem. 1982, 25, 1269-1277,
- et une fonction phénol ou naphtol, sous forme libre ou protégée.

De préférence, on utilise les rétinoïdes à fonction phénol.

Parmi les groupes protecteurs de la fonction phénol ou naphtol, on préfère un groupe éther ou ester.

Ces groupements protecteurs sont notamment décrits dans le livre : "Protective groups in organic synthesis ", by theodora W. Greene et Peter G. Wuts,Ed John Wiley and Sons, 1991.

Parmi les rétinoïdes portant une fonction phénol ou naphtol, on peut notamment citer les composés suivants :
Acide 6-[3-(1-adamantyl)-4-méthoxyphenyl]-2-naphtoique,
6-[3-(1-adamantyl)-4-hydroxyphenyl]-2-naphtoate de methyle,
Acide 6-[3-(1-adamantyl)-4-hydroxyphenyl]-2-naphtoique,
Acide 6-[3-(1-adamantyl)-4-acetoxyphenyl]-2-naphtoique,
Acide [3-[3-(1-adamantyl)-4-methoxyphenyl]-2H-1-benzopyran]-7-carboxylique,
Acide [3-[3-(1-adamantyl)-4-hydroxyphenyl]-2H-1-benzopyran]-7-carboxylique,
Acide [3-[3-(1-adamantyl)-4-acetoxyphenyl]-2H-1-benzopyran]-7-carboxylique,
Acide [2-[3-(1-adamantyl)-4-hydroxyphenyl] benzimidazole] 5-carboxylique,
Acide [2-[3-(1-adamantyl)-4-hydroxyphenyl] benzothiophene 6-carboxylique,
Acide [2-[3-(1-adamantyl)-4-hydroxyphenyl] benzofuranne 6-carboxylique,
Acide 4-[(E)-2-[3-(1-adamantyl)-4-hydroxyphenyl]propenyl]-benzoique,
Acide 4-[2-[3-(1-adamantyl)-4-hydroxyphenyl]ethynyl]-benzoique,
Acide 3-[(3,5-di-tert-Butyl-4-hydroxyphenyl)-2H-1-benzopyran]-7-carboxylique,
3-[(3,5-di-tert-Butyl-4-hydroxyphenyl)-2H-1-benzopyran]-7-carboxylate de methyle,
Acide 6-[3-tert-Butyl-4-hydroxyphenyl]naphtoïque,
Acide 4-[(E)-2-[3,5-di-tert-Butyl-4-hydroxyphenyl]ethenyl]-benzoique,
Acide 4-[(E)-2-[3,5-di-tert-Butyl-4-hydroxyphenyl)propenyl]-benzoique,
Acide 4-[3-(3,5-di-tert-butyl-4-hydroxyphényl)-1-propynyl] benzoïque,
Acide (*all*-*E*)-9-(3-(1-Adamantyl)-4-Hydroxyphenyl)-3,7-dimethyl-2,4,6-nonatrienoïque,
Acide (*all-E*)-9-(4-Hydroxy-2,3,6-trimethylphenyl)-3,7-dimethyl-2,4,6,8-nonatetraenoique,
(*all-E*)-9-(4-Methoxy-2, 3,6-trimethylphenyl)-3,7-dimethyl-2,4,6,8-nonatetraenoate d'ethyle,
Acide 4-[6-Hydroxy-7-(1-Adamantyl)-2-naphtyl]benzoïque,
Acide 4-[(E)-2-[3-tert-Butyl-4-hydroxyphenyl]propenyl]benzoïque.

Ces composés présentent respectivement les formules (1) à (23) développées suivantes :

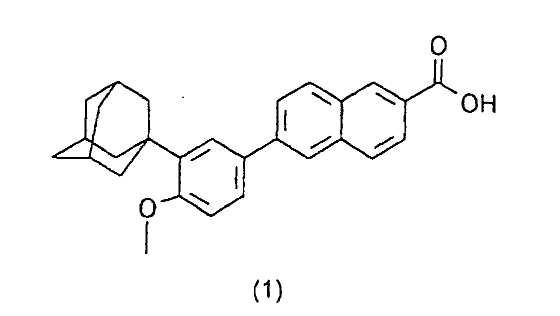

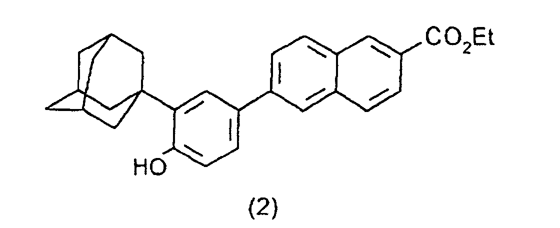

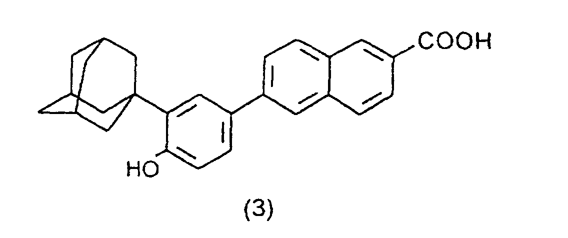

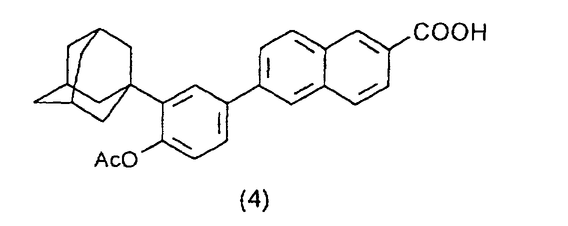

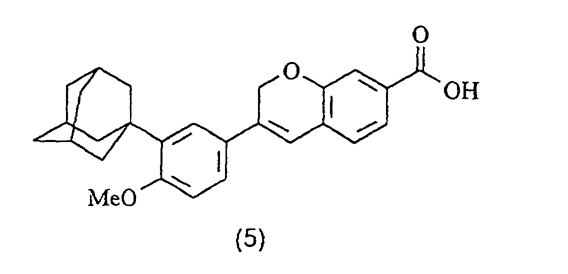

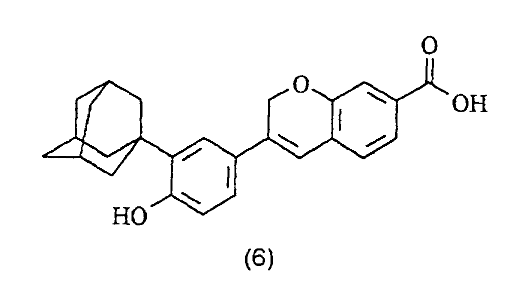

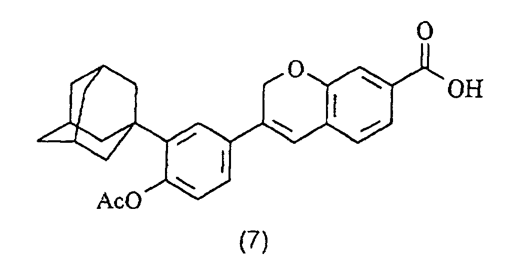

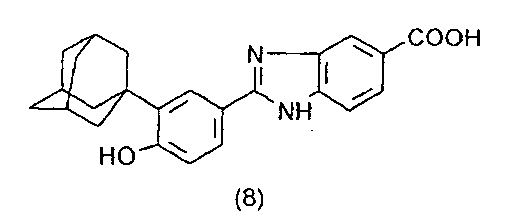

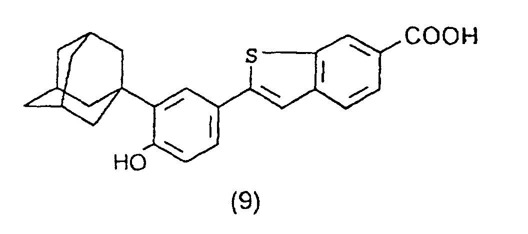

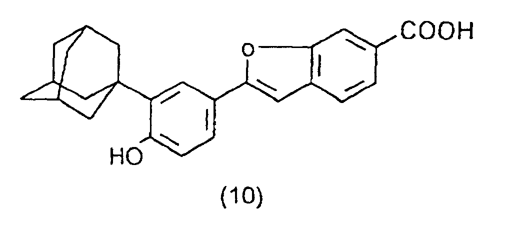

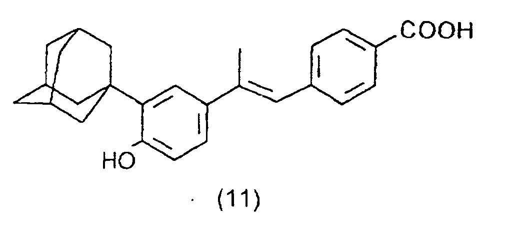

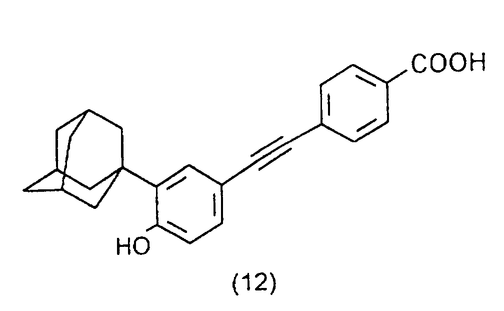

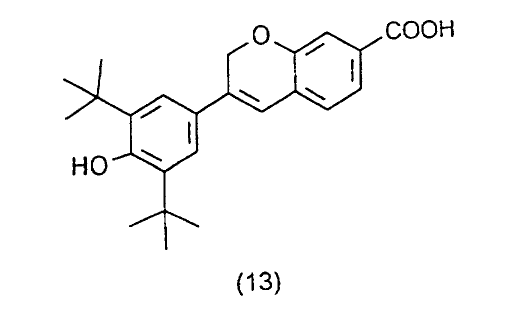

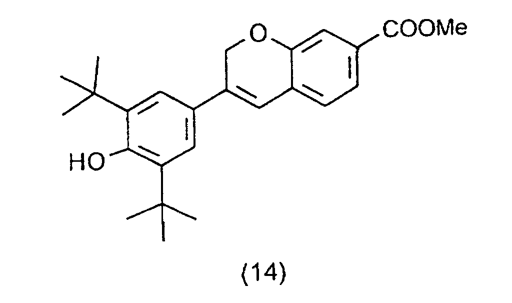

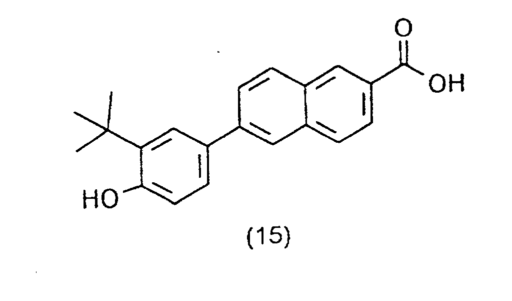

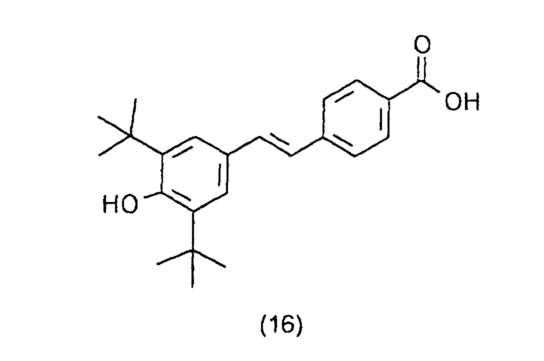

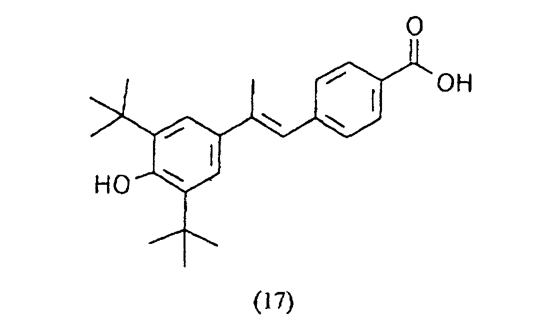

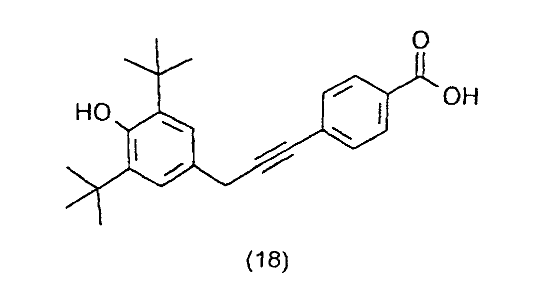

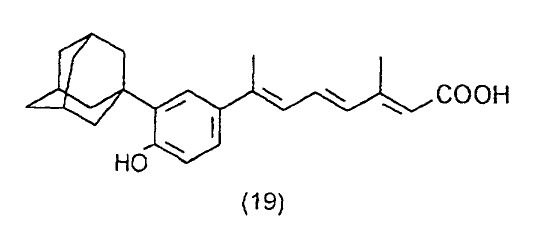

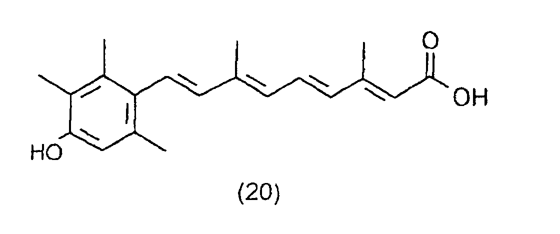

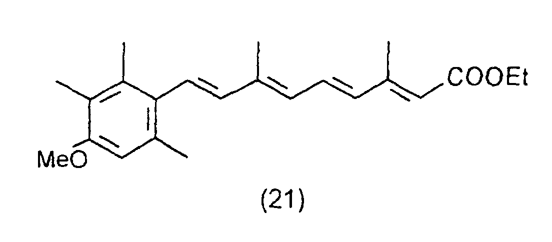

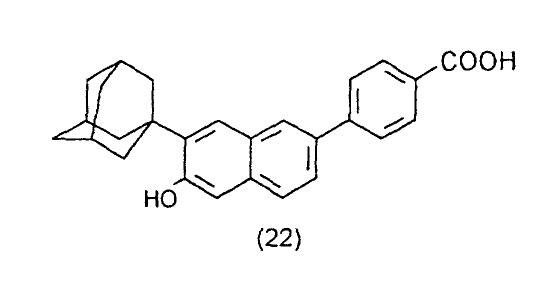

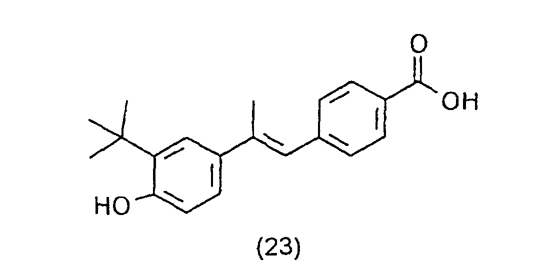

Parmi ceux-ci, on préfère utiliser les rétinoïdes portant une fonction phénol ou naphtol suivants :
Acide 6-[3-(1-adamantyl)-4-hydroxyphenyl]-2-naphtoique,
Acide 4-[(E)-2-[3-(1-adamantyl)-4-hydroxyphenyt]propenyl]-benzoique,
Acide 3-[(3,5-di-tert-Butyl-4-hydroxyphenyl)-2H-1-benzopyran]-7-carboxylique,
Acide 4-[(E)-2-[3,5-di-tert-Butyl-4-hydroxyphenyl]propenyl]-benzoique,
Acide 4-[3-(3,5-di-tert-butyl-4-hydroxyphényl)-1-propynyl] benzoïque.

Le rétinoïde selon l'invention peut être employé pour des usages médicaux ou de confort (cosmétique). Il peut être notamment employé dans le traitement des dérèglements de la pigmentation comme le vitiligo, l'albinisme, des hypopygmentations post-inflammatoires, dans le traitement des hypopygmentations ou dépigmentations après des greffes de peau, dans le traitement des hypopygmentations ou dépigmentations dues à une surexposition aux rayons ultra-violet, traiter des hypopygmentations ou dépigmentations post-cicatrisation ou de traiter des hypopygmentations ou dépigmentations dues au vieillissement ou les lentigos. Ils peuvent également être employés pour la recoloration des cheveux, plus particulièrement les cheveux gris, ou pour traiter les problèmes de pigmentation des ongles.

Un autre objet de l'invention concerne donc une composition cosmétique ou pharmaceutique comprenant, dans un support physiologiquement acceptable, au moins un rétinoïde portant une fonction phénol ou naphtol et au moins un substrat d'au moins une enzyme présentant une activité tyrosinase.

Parmi les substrats utilisables selon l'invention on peut citer par exemple la tyrosine ou ses dérivés, la dihydroxy-3,4 phényl α-alanine (DOPA) ou le 5,6-dihydroxyindole.

Il est possible de réunir en une seule composition le rétinoïde et le substrat. Mais des formes particulières de réalisation peuvent être envisagées, en particulier le rétinoïde et le substrat peuvent être administrés de manière simultanée, séparée ou étalée dans le temps.

Ainsi, l'invention a aussi pour objet une composition comprenant au moins un rétinoïde à fontion phénol ou naphtol et au moins un substrat d'au moins une enzyme présentant une activité tyrosinase comme produit de combinaison pour une utilisation simultanée, séparée ou étalée dans le temps pour favoriser la pigmentation de la peau et/ou des cheveux.

Sous une forme de réalisation particulière le rétinoïde et le substrat peuvent être conditionnés de manière séparée sous forme d'un kit dont les composants seront mélangés extemporanément.

L'invention a ainsi pour objet un kit conprenant au moins un rétinoïde à fonction phénol ou naphtol et au moins un substrat d'au moins une enzyme présentant une activité tyrosinase pour une utilisation simultanée, séparée ou étalée dans le temps pour favoriser la pigmentation de la peau et/ou de ses phanères.

La composition comprenant le rétinoïde selon l'invention peut être ingérée, injectée ou appliquée sur la peau (sur toute zone cutanée du corps) ou ses phanères, plus particulièrement les cheveux et les ongles. Selon le mode d'administration, la composition utilisée selon l'invention peut se présenter sous toutes les formes galéniques.

La quantité suffisante de rétinoïdes utilisés ou compris dans les compositions correspond bien entendu à la quantité nécessaire pour favoriser la pigmentation attendue, cette quantité dépend notamment de la qualité pigmentante du rétinoïde choisi et de la composition dans lequel il se trouve et de la fréquence à laquelle la composition comprenant le rétinoïde est utilisée.

Par voie entérale, les médicaments peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.

Pour donner un ordre de grandeur, les rétinoïdes ingérés ou injectés selon l'invention sont généralement administrés à une dose journalière d'environ 0,01 mg/kg à 100 mg/kg en poids corporel en 1 à 3 prises.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Les compositions peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

Ces compositions pour la voie topique contiennent au moins un rétinoïde portant une fonction phénol ou naphtol selon l'invention à une concentration de préférence comprise entre 0,001 et 5 % par rapport au poids total de la composition.

La concentration en rétinoïde portant une fonction phénol ou naphtol selon l'invention dans les compositions cosmétiques à usage topique est préférablement comprise entre 0,001 et 3 % en poids.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un rétinoïde à fonction phénol ou naphtol à d'autres agents actifs. Parmi ces agents actifs, on peut citer à titre d'exemple :
- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les agents favorisant la repousse des cheveux comme ceux décrits par la demanderesse dans la demande de brevet européen publiée sous le numéro 0648488 ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ,
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde, dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme le peroxyde de benzoyle ;

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétrayénoïque et eicosatriyénoïque ou leurs esters et amides, la vitamine D et ses dérivés, des extraits d'origine végétale ou bactérienne.

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

On peut également envisager que la composition comprenant au moins un rétinoïde à fonction phénol ou naphtol soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

Bien entendu, l'homme du métier veille à ne pas introduire des composés à la composition utilisée dans la présente invention d'une manière telle que ces derniers contreviennent à l'effet technique désiré, objet de la présente invention.

La composition pharmaceutique selon l'invention peut être administrée par voie parentérale, entérale ou encore par voie topique. De préférence, la composition pharmaceutique est administrée par voie topique.

On va maintenant donner à titre d'illustration des exemples qui ne sauraient limiter en aucune façon la portée de l'invention.

### EXEMPLE 1

### Activité pigmentante de rétinoïdes à fonction phénol ou naphtol

Le test utilisé est celui décrit dans la demande de brevet français n° 2734825. La synthèse de la mélanine est estimée par incorporation de thiouracile. L'activité spécifique de la mélanogénèse est estimée par le rapport de l'incorporation de ¹⁴C thiouracile à l'incorporation de ³H leucine rapporté à 100% du témoin (sans rétinoïde).

Ainsi, l'activité pigmentante est classée de la manière suivante :
classe 1 : moins de 30% d'activité spécifique de la mélanogénèse,
classe 2 : de 30% à 60% d'activité spécifique de la mélanogénèse,
classe 3 : plus de 60% d'activité spécifique de la mélanogénèse,
L'AC50 est la concentration pour laquelle on observe 50% de stimulation de la mélanogénèse par rapport au témoin.

Le tableau suivant rassemble les résultats.

| Composé de formule | Activité pigmentante | AC50 |
|---|---|---|
| (3) | classe 2 | 10⁻⁸ |
| (11) | classe 2 | 10⁻⁸-10⁻⁷ |
| (17) | classe 1 | < 10⁻¹⁰ |
| (18) | classe 3 | ND |
| (13) | classe 3 | ND |
| (16) | classe 1 | ND |
| ND : Non Déterminé | | |

A titre de comparaison, l'acide rétinoïque *tout-trans* dans les mêmes conditions présente une activité pigmentante de classe -1, c'est à dire présente une activité dépigmentante de classe 1 (moins de 30% d'activité spécifique d'inhibition de la mélanogénèse).

### EXEMPLES DE FORMULATIONS

### 1) VOIE ORALE

(a) On prépare la composition suivante sous la forme d'un comprimé de 0,8 g

| | |
|---|---|
| Composé de formule (2) | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement d'une hypopigmentation post-inflammatoire, on administre à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de formule (4) | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Pour le traitement d'une hypopigmentation après greffe de peau, on administre à un individu adulte 1 ampoule par jour pendant 3 mois selon la gravité du cas traité.
(c) On prépare la formulation suivante destinée à être conditionnée en gélules :

| | |
|---|---|
| Composé de formule (5) | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du vitiligo, on administre à un individu adulte, 1 gélule par jour pendant 30 jours.

### 2) VOIE TOPIQUE

(a) On prépare la crème Eau-dans l'Huile non ionique suivante :

| | |
|---|---|
| Composé de formule (8) | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau greffée hypopigmentée 1 à 2 fois par jour pendant 30 jours.
(b) On prépare un gel en réalisant la formulation suivante :

| | |
|---|---|
| Composé de formule (11) | 0,050 g |
| Erythromycine base | 4,000 g |
| Butylhydroxytoluéne | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau greffée hypopigmentée 1 à 3 fois par jour pendant 6 à 12 semaines selon la gravité du cas traité.
(c) On prépare une lotion pour corriger les hypopigmentation post-inflammatoire en procédant au mélange des ingrédients suivants :

| | |
|---|---|
| Composé de formule (2) | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de formule (4) | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit
(e) On prépare la crème Huile dans l'Eau non ionique suivante :

| | |
|---|---|
| Composé de formule (3) | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau atteinte de vitiligo 1 à 2 fois par jour pendant 30 Jours.
(f) On prépare un gel topique en procédant au mélange des ingrédients suivants :

| | |
|---|---|
| Composé de formule (17) | 0,050 g |
| Ethanol | 43,000 g |
| α-tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel est appliqué sur une peau atteinte de vitiligo 1 à 3 fois par jour pendant 6 à 12 semaines selon la gravité du cas traité.
(g) On prépare une lotion capillaire pour repigmenter les cheveux en procédant au mélange des ingrédients suivants :

| | |
|---|---|
| Composé de formule (9) | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une dépigmentation importante.
(h) On prépare une crème en situation de post-cicatrisation en procédant au mélange des ingrédients suivants :

| | |
|---|---|
| Composé de formule (13) | 0,050 g |
| Acide rétinoïque | 0,010 g |
| Mélange de stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée 1 à 3 fois par jour pendant 6 à 12 semaines
(i) On prépare une crème huile dans l'eau en réalisant la formulation suivante :

| | |
|---|---|
| Composé de formule (4) | 0,020 g |
| 17-valérate de bétamethasone | 0,050 g |
| S-carboxyméthyl cystéine | 3,000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000 g |

Cette crème est appliquée 2 fois par jour sur une peau atteinte de problèmes de pigmentation dus au viellissement.
(j) On prépare la crème de type huile dans l'eau suivante :

| | |
|---|---|
| Acide lactique | 5,000 g |
| Composé de formule (13) | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" , par la société "DYNAMIT NOBEL" | 4,000 g |
| Eau q.s.p | 100,000 g |

(k) Lotion dermique à pulvériser :

| | |
|---|---|
| composé de formule (15) | 5,000 g |
| Ethanol | 30,000 g |
| Eau déminéralisée | q.s.p. 100,000 g |

(I) Lotion pour les cheveux :

| | |
|---|---|
| composé de formule (18) | 3,000 g |
| Propylène glycol | 30,000 g |
| Alcool éthylique | 40,500 g |
| Eau | q.s.p. 100,000 g |

On applique cette lotion sur le cuir chevelu, une à deux fois par jour, à raison d'1 ml par application.
(m) Lotion épaissie :

| | |
|---|---|
| composé de formule (1) | 5,000 g |
| Kawaïne | 2,000 g |
| Hydroxypropylcellulose (Klucel G de la société Hercules) | 3,500 g |
| Alcool éthylique | qsp 100,000 g |

On applique cette lotion épaissie sur le cuir chevelu, une à deux fois par jour, à raison d'1 ml par application.
(n) Lotion niosomée :

| | | |
|---|---|---|
| Chimexane NL® | | 0,475 g |
| Cholestérol | | 0,475 g |
| Stéaroylglutamate monosodique | | 0,050 g |
| composé de formule (3) | | 0,100 g |
| Conservateurs | qs | |
| Colorants | qs | |
| Parfum | qs | |
| Eau déminéralisée | qsp | 100,000 g |

On applique cette lotion sur le cuir chevelu, une à deux fois par jour, à raison d'1 ml par application.
(o) Lotion :

| | |
|---|---|
| composé de formule (17) | 5,000 g |
| Monométhyléther de propylèneglycol (Dowanol PM de Dow Chemical) | 20,000 g |
| Hydroxypropylcellulose (Klucel G de la société Hercules) | 3,000 g |
| Alcool éthylique | 40,000 g |
| Minoxidil | 2,000 g |
| Eau | qsp 100,000 g |

On applique cette lotion épaissie sur le cuir chevelu, une à deux fois par jour, à raison d'1 ml par application.

## Revendications

1. Utilisation dans une composition cosmétique, à titre de principe actif, d'une quantité suffisante d'au moins un rétinoïde portant une fonction phénol ou naphtol, ce rétinoïde ou la composition étant destiné à favoriser la pigmentation de la peau et/ou de ses phanères.

2. Utilisation, à titre de principe actif, d'une quantité suffisante d'au moins un rétinoide portant une fonction naphtol ou phénol pour la préparation d'une composition pharmaceutique destinée à favoriser la pigmentation de la peau et/ou de ses phanères.

3. Utilisation selon la revendication 1 ou 2, **caractérisée en ce que** le rétinoïde portant une fonction phénol ou naphtol est choisi parmi les rétinoïdes portant une fonction phénol.

4. Utilisation selon la revendication 1 ou 2 ou 3, **caractérisée en ce que** le rétinoïde portant une fonction phénol ou naphtol est choisi parmi les rétinoïdes :
Acide 6-[3-(1-adamantyl)-4-méthoxyphenyl]-2-naphtoique,
6-[3-(1-adamantyl)-4-hydroxyphenyl]-2-naphtoate de methyle,
Acide 6-[3-(1-adamantyl)-4-hydroxyphenyl]-2-naphtoique,
Acide 6-[3-(1-adamantyl)-4-acetoxyphenyl]-2-naphtoique,
Acide [3-[3-(1-adamantyl)-4-methoxyphenyl]-2H-1-benzopyran]-7-carboxylique,
Acide [3-[3-(1-adamantyl)-4-hydroxyphenyl]-2H-1-benzopyran]-7-carboxylique,
Acide [3-[3-(1-adamantyl)-4-acetoxyphenyl]-2H-1-benzopyran]-7-carboxylique,
Acide [2-[3-(1-adamantyl)-4-hydroxyphenyl] benzimidazole] 5-carboxylique,
Acide [2-[3-(1-adamantyl)-4-hydroxyphenyl] benzothiophene 6-carboxylique,
Acide [2-[3-(1-adamantyl)-4-hydroxyphenyl] benzofuranne 6-carboxylique,
Acide 4-[(E)-2-[3-(1-adamantyl)-4-hydroxyphenyl]propenyl]-benzoique,
Acide 4-[2-[3-(1-adamantyl)-4-hydroxyphenyl]ethynyl]-benzoique,
Acide 3-[(3,5-di-tert-Butyl-4-hydroxyphenyl)-2H-1-benzopyran]-7-carboxylique,
3-[(3,5-di-tert-Butyl-4-hydroxyphenyl)-2H-1-benzopyran]-7-carboxylate de methyle,
Acide 6-[2-[3-tert-Butyl-4-hydroxyphenyl]-naphtoïque,
Acide 4-[(E)-2-[3,5-di-tert-Butyl-4-hydroxyphenyl]ethenyl]-benzoique,
Acide 4-[(E)-2-[3,5-di-tert-Butyl-4-hydroxyphenyl]propenyl]-benzoique,
Acide 4-[3-(3,5-di-tert-butyl-4-hydroxyphényl)-1 -propynyl] benzoïque,
Acide (*all-E*)-9-(3-(1-Adamantyl)-4-Hydroxyphenyl)-3,7-dimethyl-2,4,6-nonatrienoïque,
Acide (*all-E*) -9 - (4 -Hydroxy -2,3,6-trimethylphenyl)-3,7 -dimethyl -2,4,6,8-nonatetraenoique,
(*all-E*)-9-(4-Methoxy-2,3,6-trimethylphenyl)-3,7-dimethyl-2,4,6,8-nonatetraenoate d'ethyle,
Acide 4-[6-Hydroxy-7-(1-Adamantyl)-2-naphtyl]benzoïque,
Acide 4-[(E)-2-[3-tert-Butyl-4-hydroxyphenyl]propenyl]benzoïque.

5. Utilisation selon la revendication précédente, **caractérisé en ce que** le rétinoïde portant une fonction phénol ou naphtol est choisi parmi :
Acide 6-[3-(1-adamantyl)-4-hydroxyphenyl]-2-naphtoique,
Acide 4-[(E)-2-[3-(1-adamantyl)-4-hydroxyphenyl]propenyl]-benzoique,
Acide 3-[(3,5-di-tert-Butyl-4-hydroxyphenyl)-2H-1-benzopyran]-7-carboxylique,
Acide 4-[(E)-2-[3,5-di-tert-Butyl-4-hydroxyphenyl]propenyl]-benzoique,
Acide 4-[3-(3,5-di-tert-butyl-4-hydroxyphényl)-1-propynyl] benzoïque;

6. Utilisation selon l'une quelconque des revendications précédentes, **caractérisée en ce que** la composition est destinée à favoriser la pigmentation de la peau et/ou de ses phanères dans le but de traiter des dérèglements de la pigmentation comme le vitiligo, l'albinisme, des hypopygmentations post-inflammatoires, des hypopygmentations ou dépigmentations après des greffes de peau, des hypopygmentations ou dépigmentations dues à une surexposition aux rayons ultra-violet, traiter des hypopygmentations ou dépigmentations post-cicatrisation, des hypopygmentations ou dépigmentations dues au vieillissement de traiter les lentigos ou de traiter les problèmes de pigmentation des ongles.

7. Utilisation selon l'une quelconque des revendications précédentes, **caractérisée en ce que** la composition comprenant le rétinoïde peut être ingérée, injectée ou appliquée sur la peau ou ses phanères, plus particulièrement les cheveux et les ongles.

8. Utilisation selon la revendication précédente, **caractérisée en ce que** la composition appliquée sur la peau ou ses phanères comprend au moins un rétinoïde portant une fonction phénol ou naphtol à une concentration de préférence comprise entre 0,001 et 5 % par rapport au poids total de la composition.

9. Procédé cosmétique pour pigmenter la peau et/ou ses phanères, **caractérisé en ce que** l'on applique sur la peau et/ou ses phanères une composition comprenant, dans un support cosmétiquement acceptable, au moins un rétinoïde portant une fonction phénol ou naphtol tel que défini dans l'une quelconque des revendications 1 à 5.

10. Composition cosmétique ou pharmaceutique comprenant, dans un support physiologiquement acceptable, au moins un rétinoïde portant une fonction phénol ou naphtol tel que défini dans l'une quelconque des revendications 1 à 5 et au moins un substrat d'au moins une enzyme présentant une activité tyrosinase.

11. Composition comprenant au moins un rétinoïde à fontion phénol ou naphtol tel que défini dans l'une quelconque des revendications 1 à 5 et au moins un substrat d'au moins une enzyme présentant une activité tyrosinase comme produit de combinaison pour une utilisation simultanée, séparée ou étalée dans le temps pour favoriser la pigmentation de la peau et/ou des cheveux

12. Kit comprenant au moins un rétinoïde à fonction phénol ou naphtol tel que défini dans l'une quelconque des revendications 1 à 5 et au moins un substrat d'au moins une enzyme présentant une activité tyrosinase pour une utilisation simultanée, séparée ou étalée dans le temps pour favoriser la pigmentation de la peau et/ou de ses phanères.

13. Composition selon l'une des revendications précédentes 10 à 12, **caractérisée en ce que** le substrat d'au moins une enzyme présentant une activité tyrosinase est choisi parmi la tyrosine ou ses dérivés, la dihydroxy-3,4 phényl α-alanine et le 5,6-dihydroxyindole.

## Patentansprüche

1. Verwendung mindestens eines Retinoids, das eine Phenol- oder Naphtholgruppe aufweist, in einer ausreichenden Menge als Wirkstoff in einer kosmetischen Zusammensetzung, wobei das Retinoid oder die Zusammensetzung dazu vorgesehen sind, die Pigmentierung der Haut und/oder der Hautanhangsgebilde zu stimulieren.

2. Verwendung mindestens eines Retinoids, das eine Phenol- oder Naphtholgruppe aufweist, in einer ausreichenden Menge als Wirkstoff zur Herstellung einer pharmazeutischen Zusammensetzung, die dazu vorgesehen ist, die Pigmentierung der Haut und/oder der Hautanhangsgebilde zu stimulieren.

3. Verwendung nach Anspruch 1 oder 2, **dadurch gekennzeichnet, daß** das Retinoid, das eine Phenol- oder Naphtholgruppe aufweist, unter den Retinoiden mit Phenolgruppe ausgewählt ist.

4. Verwendung nach Anspruch 1, 2 oder 3, **dadurch gekennzeichnet, daß** das Retinoid, das eine Phenol- oder Naphtholgruppe aufweist, unter den folgenden Verbindungen ausgewählt ist:
6-[3-(1-Adamantyl)-4-methoxyphenyl]-2-naphthoesäure,
Methyl-6-[3-(1-adamantyl)-4-hydroxyphenyl]-2-naphthoat,
6-[3-(1-Adamantyl)-4-hydroxyphenyl]-2-naphthoesäure,
6-[3-(1-Adamantyl)-4-acetoxyphenyl]-2-naphthoesäure,
[3-[3-(1-Adamantyl)-4-methoxyphenyl]-2H-1-benzopyran]-7-carbonsäure,
[3-[3-(1-Adamantyl)-4-hydroxyphenyl]-2H-1-benzopyran]-7-carbonsäure,
[3-[3-(1-Adamantyl)-4-acetoxyphenyl]-2H-1-benzopyran]-7-carbonsäure,
[2-[3-(1-Adamantyl)-4-hydroxyphenyl]-benzimidazol]-5-carbonsäure,
[2-(3-(1-Adamantyl)-4-hydroxyphenyl]-benzothiophen]-6-carbonsäure,
[2-[3-(1-Adamantyl)-4-hydroxyphenyl]-benzofuran]-6-carbonsäure,
4-[(E)-2-[3-(1-Adamantyl)-4-hydroxyphenyl]-propenyl]-benzoesäure,
4-[2-[3-(1-Adamantyl)-4-hydroxyphenyl]-ethinyl]-benzoesäure,
3-1(3,5-Di-*t*-butyl-4-hydroxyphenyl]-2H-1-benzopyran]-7-carbonsäure,
Methyl-3-[(3,5-di-*t*-butyl-4-hydroxyphenyl]-2H-1-benzopyran]-7-carboxylat,
6-{3-*t*-Butyl-4-hydroxyphenyl]-2-naphthoesäure,
4-[(E)-2-[3,5-Di-*t*-butyl-4-hydroxyphenyl]-ethenyl]-benzoesäure,
4-[(E)-2-[3,5-Di-*t*-butyl-4-hydroxyphenyl]-propenyl]-benzoesäure,
4-[3-[3,5-Di-*t*-butyl-4-hydroxyphenyl]-1-propinyl]-benzoesäure,
(*all*-E)-9-[3-(1-Adamantyl)-4-hydroxyphenyl]-3,7-dimethyl-2,4,6-nonatriensäure,
(*all* -E ) -9 - [4 -Hydroxy -2,3,6 -trimethylphenyl ] -3,7 -dimethyl -2,4,6,8-nonatetraensäure,
(*all*-E)-9-[4-Methoxy-2,3,6-trimethylphenyl]-3,7-dimethyl-2,4,6,8-nonatetraensäureethylester,
4-[6-Hydroxy-7-(1-Adamantyl)-2-naphthyl]-benzoesäure, und
4-[(E)-2-[3-*t*-Butyl-4-hydroxyphenyl]propenyl]-benzoesäure.

5. Verwendung nach dem vorhergehenden Anspruch, **dadurch gekennzeichnet, daß** das Retinoid mit Phenol- oder Naphtholgruppe ausgewählt ist unter:
6-[3-(1-Adamantyl)-4-hydroxyphenyl]-2-naphthoesäure,
4-[(E)-2-[3-(1-Adamantyl)-4-hydroxyphenyl]-propenyl]-benzoesäure,
3-[(3,5-Di-t-Butyl-4-hydroxyphenyl]-2H-1-benzopyran]-7-carbonsäure,
4-[(E)-2-[3,5-di-*t*-Butyl-4-hydroxyphenyl]-propenyl]-benzoesäure, und
4-[3-[3,5-Di-*t*-butyl-4-hydroxyphenyl]-1-propinyl]-benzoesäure.

6. Verwendung nach einem der vorhergehenden Ansprüche, **dadurch gekennzeichnet, daß** die Zusammensetzung dazu vorgesehen ist, die Pigmentierung der Haut und/oder der Hautanhangsgebilde zu stimulieren, mit dem Ziel, Pigmentierungsstörungen, wie Vitiligo, Albinismus und nach Entzündungen auftretende Hypopigmentierungen, Hypopigmentierungen oder Depigmentierungen nach Hauttransplantationen, Hypopigmentierungen oder Depigmentierungen, die von übermäßiger UV-Exposition herrühren, nach Narbenbildung auftretende Hypopigmentierungen oder Depigmentierungen, Hypopigmentierungen oder Depigmentierungen, die mit der Alterung oder zusammenhängen, Leberflecken oder Pigmentierungsprobleme an den Nägeln zu behandeln.

7. Verwendung nach einem der vorhergehenden Ansprüche, **dadurch gekennzeichnet, daß** die Zusammensetzung, die das Retinoid enthält, eingenommen, injiziert oder auf die Haut oder die Hautanhangsgebilde, insbesondere die Haare oder die Nägel, aufgetragen werden kann.

8. Verwendung nach dem vorhergehenden Anspruch, **dadurch gekennzeichnet, daß** die auf die Haut und/oder die Hautanhangsgebilde aufgebrachte Zusammensetzung mindestens ein Retinoid mit Phenol- oder Naphtholgruppe in einer Konzentration vorzugsweise von 0,001 bis 5 %, bezogen auf das Gesamtgewicht der Zusammensetzung, enthält.

9. Kosmetisches Verfahren zur Pigmentierung der Haut und/oder der Hautanhangsgebilde, **dadurch gekennzeichnet, daß** auf die Haut und/oder die Hautanhangsgebilde eine Zusammensetzung aufgebracht wird, die mindestens ein Retinoid mit Phenol- oder Naphtholgruppe nach der in einem der Ansprüche 1 bis 5 angegebenen Definition enthält.

10. Kosmetische oder pharmazeutische Zusammensetzung, die in einem physiologisch akzeptablen Träger mindestens ein Retinoid mit Phenol- oder Naphtholgruppe nach der in einem der Ansprüche 1 bis 5 angegebenen Definition und ein Substrat mindestens eines Enzyms, das eine Tyrosinase-Aktivität besitzt, enthält.

11. Zusammensetzung, die mindestens ein Retinoid mit Phenol- oder Naphtholgruppe nach der in einem der Ansprüche 1 bis 5 angegebenen Definition und mindestens ein Substrat mindestens eines Enzyms, das eine Tyrosinase-Aktivität besitzt, enthält, als Kombinationsprodukt für eine gemeinsame, getrennte oder zeitlich aufeinanderfolgende Verwendung, um die Pigmentierung der Haut und/oder der Hautanhangsgebilde zu stimulieren.

12. Kit, der mindestens ein Retinoid mit Phenol- oder Naphtholgruppe nach der in einem der Ansprüche 1 bis 5 angegebenen Definition und mindestens ein Substrat mindestens eines Enzyms, das eine Tyrosinase-Aktivität besitzt, enthält, als Kombinationsprodukt für eine gemeinsame, getrennte oder zeitlich aufeinanderfolgende Verwendung, um die Pigmentierung der Haut und/oder der Hautanhangsgebilde zu stimulieren.

13. Zusammensetzung nach einem der Ansprüche 10 bis 12, **dadurch gekennzeichnet, daß** das Substrat eines Enzyms, das eine Tyrosinase-Aktivität besitzt, unter Tyrosin oder seinen Derivaten, α-(3,4-Dihydroxyphenyl)-alanin oder 5,6-Dihydroxyindol ausgewählt ist.

## Claims

1. Use, in a cosmetic composition, as active principle, of a sufficient amount of at least one retinoid bearing a phenol or naphthol function, this retinoid or the composition being intended to promote pigmentation of the skin and/or its integumentes.

2. Use, as active principle, of a sufficient amount of at least one retinoid bearing a naphthol or phenol function for the preparation of a pharmaceutical composition intended to promote pigmentation of the skin and/or its integumentes.

3. Use according to Claim 1 or 2, **characterized in that** the retinoid bearing a phenol or naphthol function is chosen from retinoids bearing a phenol function.

4. Use according to Claim 1 or 2 or 3, **characterized in that** the retinoid bearing a phenol or naphthol function is chosen from the following retinoids:
6-[3-(1-adamantyl)-4-methoxyphenyl]-2-naphthoic acid,
methyl 6-[3-(1-adamantyl)-4-hydroxyphenyl]-2-naphthoate,
6-[3-(1-adamantyl)-4-hydroxyphenyl]-2-naphthoic acid,
6-[3-(1-adamantyl)-4-acetoxyphenyl]-2-naphthoic acid,
[3-[3-(1-adamantyl)-4-methoxyphenyl]-2H-1-benzopyran]-7-carboxylic acid,
[3-[3-(1-adamantyl)-4-hydroxyphenyl]-2H-1-benzopyran]-7-carboxylic acid,
[3-[3-(1-adamantyl)-4-acetoxyphenyl]-2H-1-benzopyran]-7-carboxylic acid,
[2- [3-(1-adamantyl)-4-hydroxyphenyl]benzimidazole]-5-carboxylic acid,
[2- [3-(1-adamantyl)-4-hydroxyphenyl]benzothiophene-6-carboxylic acid,
[2-[3- (1-adamantyl)-4-hydroxyphenyl]benzofuran-6-carboxylic acid,
4-[(E)-2-[3-(1-adamantyl)-4-hydroxyphenyl]propenyl]-benzoic acid,
4-[2-[3-(1-adamantyl)-4-hydroxyphenyl]ethynyl]benzoic acid,
3-[(3,5-di-tert-butyl-4-hydroxyphenyl)-2H-1-benzopyran]-7-carboxylic acid,
methyl 3-[(3,5-di-tert-butyl-4-hydroxyphenyl) -2H-1-benzopyran]-7-carboxylate,
6-[2-[3-tert-butyl-4-hydroxyphenyl]naphthoic acid,
4-[(E)-2-[3,5-di-tert-butyl-4-hydroxyphenyl]ethenyl]-benzoic acid,
4-[(E)-2-[3,5-di-tert-butyl-4-hydroxyphenyl]propenyl]-benzoic acid,
4-[3-(3,5-di-tert-butyl-4-hydroxyphenyl)-1-propynyl]-benzoic acid,
(all-E) -9- (3-(1-adamantyl)-4-hydroxyphenyl)-3,7-dimethyl-2,4,6-nonatrienoic acid,
(all-E)-9-(4-hydroxy-2,3,6-trimethylphenyl)-3,7-dimethyl-2,4,6,8-nonatetraenoic acid,
ethyl (all-E)-9-(4-methoxy-2,3,6-trimethylphenyl)-3,7-dimethyl-2,4,6,8-nonatetraenoate,
4-[6-hydroxy-7-(1-adamantyl) -2-naphthyl]benzoic acid,
4-[(E)-2-[3-tert-butyl-4-hydroxyphenyl)propenyl]benzoic acid.

5. Use according to the preceding claim, **characterized in that** the retinoid bearing a phenol or naphthol function is chosen from:
6-[3-(1-adamantyl)-4-hydroxyphenyl]-2-naphthoic acid,
4-[(E)-2-[3-(1-adamantyl)-4-hydroxyphenyl]propenyl]-benzoic acid,
3-[(3,5-di-tert-butyl-4-hydroxyphenyl)-2H-1-benzopyran]-7-carboxylic acid,
4-[(E)-2-[3,5-di-tert-butyl-4-hydroxyphenyl]propenyl]-benzoic acid,
4-[3-(3,5-di-tert-butyl-4-hydroxyphenyl)-1-propynyl]-benzoic acid.

6. Use according to any one of the preceding claims, **characterized in that** the composition is intended to promote pigmentation of the skin and/or its integumentes with the aim of treating pigmentation disturbances such as vitiligo, albinism, post-inflammatory hypopigmentations, hypopigmentations or depigmentations after skin grafts, hypopigmentations or depigmentations due to overexposure to ultraviolet rays, for treating post-cicatrization hypopigmentations or depigmentations or for treating hypopigmentations or depigmentations due to ageing or for treating lentigo or nail pigmentation problems.

7. Use according to any one of the preceding claims, **characterized in that** the composition comprising the retinoid can be ingested, injected or applied to the skin or its integumentes, more particularly the hair and the nails.

8. Use according to the preceding claim, **characterized in that** the composition applied to the skin or its integumentes comprises at least one retinoid bearing a phenol or naphthol function at a concentration preferably of between 0.001 and 5% relative to the total weight of the composition.

9. Cosmetic process for pigmenting the skin and/or its integumentes, **characterized in that** a composition comprising, in a cosmetically acceptable support, at least one retinoid bearing a phenol or naphthol function, as defined in any one of Claims 1 to 5, is applied to the skin and/or its integumentes.

10. Cosmetic or pharmaceutical composition comprising, in a physiologically acceptable support, at least one retinoid bearing a phenol or naphthol function as defined in any one of Claims 1 to 5 and at least one substrate of at least one enzyme having tyrosinase activity.

11. Composition comprising at least one retinoid containing a phenol or naphthol function as defined in any one of Claims 1 to 5, and at least one substrate of at least one enzyme having tyrosinase activity, as a combination product for simultaneous or separate use or for use spread out over time in order to promote pigmentation of the skin and/or the hair.

12. Kit comprising at least one retinoid containing a phenol or naphthol function as defined in any one of Claims 1 to 5, and at least one substrate of at least one enzyme having tyrosinase activity, for simultaneous or separate use or for use spread out over time in order to promote pigmentation of the skin and/or its integumentes.

13. Composition according to any one of the preceding Claims 10 to 12, **characterized in that** the substrate of at least one enzyme having tyrosinase activity is chosen from tyrosine or derivatives thereof, 3,4-dihydroxyphenyl-a-alanine and 5,6-dihydroxyindole.
